# EUROPEAN PATENT APPLICATION

(11) **EP 1 685 852 A1**
(43) Date of publication of application: **02.08.2006**
(21) Application number: 05290223.6
(22) Date of filing: 01.02.2005
(51) Int. Cl.: A61L 2/00, A61M 1/02, A61J 1/00, A61J 1/14

(54) **Set of disposable bags for viral inactivation of biological fluids**

(71) Applicant: Fondation pour la Recherche Diagnostique, 1785 Cressier s/Murat (CH)
(72) Inventor: Burnouf, Thierry, 59249 Aubers (FR); El-Ekiaby, Magdy, Maadi Cairo (EG); Goubran, Hadi Alphonse, Maadi Cairo (EG); Radosevich, Miryana, 59249 Aubers (FR)
(74) Representative: Touati, Catherine

(57) **Abstract**

The invention relates to a set system of disposable bags comprising a viral inactivation bag (1), comprising an inner compartment (4), an inlet facility (5) and an outlet facility (6), which are both connected to the inner compartment (4), the bag (1) being characterised in that the inner compartment (4) has an ovoid cross section, and optionally, at least one funnel bag (9), and/or a column chromatography bag (15), the different bags being connectable with each other, and to the use of said set system of disposable bags for the viral inactivation of biological fluids with excellent protein recovery.

## Description

The present invention concerns a set system of disposable bags comprising a viral inactivation bag and a method in which the set of disposable bags is used, optionally in combination with other bags and devices for further purification and manufacture of therapeutic proteins.

The invention relates to viral inactivation of biological fluids, under aseptic conditions, such as blood plasma, blood serum and plasma fractions in the form of single units or small pools.

Blood plasma and plasma fractions play an important role in modern medicine. They are mainly used for transfusion purposes in the treatment of bleeding episodes or for prophylaxis or for pre-treating patients prior to surgery in certain clinical situations.

A major drawback in the use of blood plasma or plasma fractions is the risk of transmission of blood borne viruses, such as human immunodeficiency virus (HIV), hepatitis B virus (HBV), and hepatitis C virus (HCV).

Therefore, different methods for viral inactivation of blood plasma and/or plasma fractions have been developed. For instance, the treatment with methylene blue followed by irradiation with visible light is a newly developed method for viral inactivation of blood plasma, which can be applied to single donations. Unfortunately, this method leads to some loss in protein activity, especially for factor VIII, fibrinogen and also Von Willebrand factor (VWF), and protein denaturation may be encountered at higher doses of methylene blue.

U.S. patent Nos. 4,481,189 and 4,540,573 describe the use of organic solvent/detergent combinations, or solvent alone to reduce by several orders of magnitude the infectivity of hepatitis viruses or other enveloped viruses contained in plasma and plasma products or added thereto.

U.S. patent No. 4,789,545 discloses a method for removal of organic solvent/detergent combinations, or solvent alone, from plasma and plasma products by partition into innocuous natural oils or synthetic triglycerides.

U.S. patent No. 5,094,960 describes the removal of solvent/detergent combinations, from plasma or plasma products by hydrophobic interaction chromatography under mild conditions, i.e. at the native concentrations of proteins, salts and other physiologic constituents.

Solvent/detergent or solvent alone treatment under appropriate conditions of reagent concentration, temperature and contact time effectively disassembles viruses that have envelope proteins associated with lipids, while having negligible effect on the molecular conformations and biological activity of sensitive plasma proteins.

However, the viral inactivation of human plasma by solvent/detergent, or solvent alone treatment has always involved the treatment of large pools of a large number of donations (100 to 500 litres, or more) and has required large and expensive pharmaceutical facilities.

Moreover, the solvent/detergent, or solvent alone, treatment is not effective against non-enveloped viruses, such as the currently known plasma-borne viruses parvovirus B19 or hepatitis A virus (HAV). Therefore, if one of the donations of the plasma pool is contaminated by a non-enveloped virus the whole pool will be contaminated.

In addition, it has been shown, for reasons that are not clarified but could be due to the harsh industrial processing steps performed at a large scale, that the industrial solvent/detergent treatment, as applied to large pools of plasma, leads to a loss of protease inhibitor and antithrombotic/anticoagulant proteins such as Protein S and alpha-1 antiplasmin, as well as in a reduction of the high-molecular-weight multimers of Von Willebrand Factor.

One of the objectives of the present invention is to provide for a convenient, easy- to-use and cost-effective means that allows for the viral inactivation of small pools of biological fluids, such as blood plasma or plasma fractions. It is a further object to avoid protein denaturation and loss in protein activity.

The inventors have now found surprisingly and after intensive research, that the objective can be reached with a set of disposable bags comprising a viral inactivation bag, comprising an inner compartment, an inlet facility and an outlet facility, which are both connected to the inner compartment, the shape of the bag being characterised in that the inner compartment has an ovoid cross section, and optionally this bag can be connected to other processing devices such as a set of one or several funnel bags, and/or various protein adsorption bags, and/or various protein processing bags, and/or various chromatographic bag or column or device system, the different bags or processing systems being connectable with each other to maintain bacterial sterility during the processing step and reduce the needs for bacterial filtration steps that induce protein losses.

Preferably the ovoid cross section of the viral inactivation bag is elliptic.

The ovoid, preferably elliptic cross section of the inner compartment has the advantage of not having any angles, which could hold back some of the biological fluid and avoid the total extensive mixing of the fluid with the viral inactivating agents.

The viral inactivation bag is made of a therapeutically acceptable and flexible material. By flexible material is understood a material that can be deformed when submitted to pressure or stress, for example due to filling and shaking the bag. The material should maintain its flexibility at temperatures in the range of 5°C to 60°C, most particularly in the range of 20 to 37°C that is commonly used to carry out viral inactivation treatment by the solvent/detergent combination or solvent alone.

A suitable material is a material which is suitable for contact with blood and plasma products and derivatives thereof, for example conventional medical/pharmaceutical grade polyvinyl chloride (PVC), as used in the blood bank and plasma industry.

The viral inactivation bag is preferably made of two laminated sheets of the therapeutically acceptable and flexible material, the two sheets being welded together on their periphery in order to form an ovoid inner compartment comprising two apertures for the inlet and outlet facilities, respectively. The welding should resist the pressure and stress produced by the biological fluid, especially when filling and shaking the bag.

In a particular embodiment the viral inactivation bag is equipped with means for suspending the bag vertically the outlet facility showing downwards. Advantageously the means for suspending the bag are arranged in the region of the upper periphery of the bag so that the bag can be hung up or laid without deformation. It can also be equipped with means for lying it horizontally (e.g. for the viral inactivation step under controlled temperature and mild shaking)

The volume of the inner compartment depends on the quantity of biological fluid to be treated. Advantageously, the volume of the inner compartment is in the range from 50 ml to 5000 ml, preferably in the range from 100 ml to 3000 ml, and even more preferably in the range from 200 ml to 2000 ml depending upon the plasma or plasma fraction to be virally-inactivated or the number of plasma units used as the starting pool.

The inlet facility and the outlet facility are preferably arranged on opposite sides of the viral inactivation bag.

In a preferred embodiment, the inlet facility comprises two ports, a first port for the transfer of the biological fluid and a second port for the addition of process chemicals. By process chemical is understood any chemical product used during viral inactivation. Advantageously, the first port is provided with a state of the art bag spike in order to allow for an easy transfer of the biological fluid from a storage or collection bag into the viral inactivation bag.

The outlet facility allows the transfer from the viral inactivation bag to a disposable funnel bag directly or by means of a flexible tube.

The disposable funnel bag used for the removal of the viral inactivating agent(s) comprises an inner compartment, an inlet facility and an outlet facility, which are both connected to the inner compartment, characterized in that the inner compartment has a lower portion in the form of a funnel that meets the outlet facility and the inner compartment contains a separation agent.

The lower portion of the inner compartment in the form of a funnel has the advantage of allowing a satisfactory, i.e. a regular and constant draining of the content and a well-controlled, potentially machine-assisted separation of biological fraction phase (bottom layer) from the oily phase (top layer).

The funnel bag is made, of a therapeutically acceptable and flexible material. By flexible material is understood a material that can be deformed when submitted to pressure or stress, for example due to filling and shaking the bag. The material should maintain its flexibility at temperatures in the range of 5°C to 60°C, most particularly in the range of 20 to 37°C that is the optimal temperature for stability of protein solutions in the absence of heat-stabilizers.

A suitable material is for example conventional polyvinyl chloride (PVC) for medical/pharmaceutical use.

The funnel bag can be made of two sheets of the therapeutically acceptable and flexible material that are put congruently on top of each other and welded together on their periphery so that the inner compartment comprising two apertures for the inlet and outlet facilities respectively is formed. The welding should resist the pressure and stress produced by the biological fluid, especially when filling and shaking the bag. Preferably the sheets are welded together by ultrasonic sealing in order to avoid contamination of the inner compartment with chemical products.

Advantageously the funnel bag comprises one at least semi-transparent portion adjacent to the outlet facility. Preferably the whole bag is transparent.

In a particular embodiment the funnel bag is equipped with means for suspending the bag vertically, the outlet facility showing downwards. Advantageously the means for suspending the bag are arranged in the region of the upper periphery of the bag so that the bag can be hung up without deformation.

The volume of the inner compartment depends essentially on the volume of the process chemicals containing biological fluid. Advantageously, the volume of the inner compartment is 10% larger than that of the viral inactivation bag, and in the range from 55 ml to 5500 ml, preferably in the range from 110 ml to 3300 ml, and even more preferably in the range from 220 ml to 2200 ml depending upon the plasma or plasma fraction to be virally-inactivated or the number of plasma units used as the starting pool.

The inlet facility and the outlet facility are preferably arranged on opposite sides of the extraction bag.

Advantageously, the inlet facility comprises a state of the art plasma bag spike in order to allow for an easy transfer of the mixture into the extraction bag. For this purpose, the outlet facility of the viral inactivation bag is punctured with the spike and the mixture is transferred into the extraction bag by gravity.

The outlet facility is preferably sealed.

The funnel bag is adapted to contain a sterile pharmaceutical oil grade in order to perform a phase separation.

The pharmaceutical grade oil can be a naturally occurring oil, for example extracted from a plant or an animal, or a synthetic compound of similar structure. Suitable naturally occurring oils include castor oil (also known as ricinus oil), soybean oil, sunfflower oil, cottonseed oil. A preferred synthetic compound is a synthetic triglyceride. Examples of suitable synthetic triglycerides include triolein, tristearin, tripalmitin, trimyristin, and combinations thereof.

The amount of pharmaceutical grade oil which is present in the bag is the amount that allows the extraction of at least 80% of lipid soluble process chemicals, the oil being used in an amount from 2 to 20 weight% based on the weight of the biological fluid, preferably from 5 to 15 weight% and more preferably from 5 to 10 weight%.

The extraction process could work with 2% or less, but the extraction procedure would have to be repeated more times, which is not preferred because it is time consuming and can cause loss of matter.

The above-described funnel bag can also be used with a chromatographic stationary phase. In this case, other forms of disposable bags can also be contemplated. When the stationary chromatographic phase has been treated in said disposable bag, it is transferred to a disposable column chromatography device.

Suitable stationary phases that can be used for further processing of the biological products comprise reversed-phase (hydrophobic interaction) matrices as those used for removal of the solvent or detergent viral inactivating agents, or protein adsorption matrices such as ion-exchange (anion and cation) matrices and affinity (such as immuno-affinity or immobilized heparin) matrices, or size-exclusion matrices. Preferred reversed phase matrices are a C18 silica packing material, or a SDR (solvent-detergent removal) hyper D. Preferred anion exchange matrices are, depending upon the protein to be purified, anion-exchangers gels, such as DEAE-Sephadex A-50, DEAE-Sepharose FF, Q-Sepharose, DEAE-Toyopearl 650M, DEAE-Hyper D.

For economic reasons, inexpensive stationary phases will be preferentially used in disposable bags according to the invention in order to fill disposable column chromatography bag devices. More expensive stationary phases are preferably recycled and thus used in traditional chromatographic columns which can be connected to disposable bags according to the invention.

The disposable column chromatography bag device can be used most particularly for the purification of the prothrombin complex concentrate on using DEAE-Sephadex A-50.

The column chromatography bag is made, of a therapeutically acceptable and semi-rigid material. By semi-rigid material is understood a material that can be deformed when submitted to pressure or stress, for example due to filling and shaking the bag. The material should maintain its semi-rigidity at temperatures in the range of 4°C to 50°C.

In a particular embodiment the bags of the set of disposable bags according to the invention are connected with each other, for example with flexible tubes. The tubes are advantageously equipped with means for stopping and eventually regulating the flow of biological fluid from one bag to another, such as clamps or valves.

In an embodiment for the viral inactivation of whole plasma used for transfusion, the set of disposable bags comprises a viral inactivation bag according to the invention, 1 to 3, preferably 2, and more preferably 3, advantageously subsequent, funnel bags according to the invention pre-filled with pharmaceutical grade oil, and a funnel bag according to the invention pre-filled with a stationary phase for column chromatography. Preferably the bags are connected among each other, i.e. the outlet facility of the viral inactivation bag is connected to the inlet facility of the first funnel bag, the outlet facility of the first funnel bag is connected to the inlet facility of the second funnel bag, the outlet facility of the second funnel bag to the inlet facility of the third funnel bag. The outlet facility of the third funnel bag is connected to a chromatographic system. Such chromatographic system is also comprised of plastic bags containing purification buffers or solvents.

In another embodiment for the viral inactivation of whole plasma used for transfusion, the set of disposable bags comprises a viral inactivation bag according to the invention, 1 to 3, preferably 2, and more preferably 3, advantageously subsequent, funnel bags according to the invention pre-filled with pharmaceutical grade oil. Preferably the bags are connected among each other, i.e. the outlet facility of the viral inactivation bag is connected to the inlet facility of the first funnel bag, the outlet facility of the first funnel bag is connected to the inlet facility of the second funnel bag, the outlet facility of the second funnel bag to the inlet facility of the third funnel bag. The outlet facility of the third funnel bag is connected to the inlet facility of a fourth bag which does not need to have funnel type shape.

In an embodiment for the viral inactivation of cryo-poor plasma used for the purification of PCC, or any other proteins present in the cryo-poor plasma, the set of disposable bags comprises a viral inactivation bag according to the invention, 1 to 3, preferably 2, and more preferably 3, advantageously subsequent, funnel bags according to the invention pre-filled with pharmaceutical grade oil, and a funnel bag according to the invention pre-filled with a stationary phase for column chromatography. Preferably the bags are connected among each other, i.e. the outlet facility of the viral inactivation bag is connected to the inlet facility of the first funnel bag, the outlet facility of the first funnel bag is connected to the inlet facility of the second funnel bag, the outlet facility of the second funnel bag to the inlet facility of the third funnel bag and the outlet facility of the third funnel bag to the inlet facility of the stationary phase containing funnel bag. The outlet facility of the stationary phase containing funnel bag is sealed. Preferably the bags are connected with flexible tubes. The tubes are advantageously equipped with means for stopping and eventually regulating the flow of biological fluid from one bag to another, such as clamps or valves.

The set of disposable bags according to the invention is very flexible in its use. It is space saving, easily transportable and therefore can be employed, wherever viral inactivation of biological fluids may be necessary. In order to use the set of disposable bags it is not necessary to dispose of any industrial facility such as a pharmaceutical factory.

Furthermore the composition of the set of disposable bags can easily be modified. It can be used with or without funnel bags and/or with or without a column chromatography bag device in order to adjust best to the required manufacturing process. The number of funnel bags can also be varied. Therefore, the set of bags can be adapted to the individual needs and requirements of every case.

Another advantage of the set of bags according to the invention is that they are discarded after each use. Thus the risk of contamination of other batches is avoided.

The invention also relates to a method in which the set of disposable bags described above is used. It particularly relates to a method of viral inactivation of a biological fluid comprising the steps of
A. viral inactivation of the biological fluid in a viral inactivation bag according to the invention,
   and optionally
B. oil extraction of the biological fluid in a pharmaceutical grade oil containing funnel bag according to the invention , and/or
C. column chromatography of the biological fluid in a column chromatography bag according to the invention.

Biological fluids according to the present invention comprise mammalian blood, blood plasma, blood serum, plasma fractions, precipitates from blood fractions and supernatants from blood fractionation, platelet poor plasma. Preferred biological fluids are blood plasma, including recovered plasma (plasma from whole blood) and apheresis plasma, plasma fractions, such as fractions for the purification of factor VIII, Von Willebrand factor, fibrinogen, fibronectin, prothrombin complex, Factor IX, Factor VII, Protein C, Protein S, Antithrombin, Alpha 1 antitrypsin, C1-inhibitor, immunoglobulins, albumin, precipitates from plasma, such as cryoprecipitate, polyethylene glycol, caprylic acid, or ammonium sulphate precipitated fractions, and their corresponding supernatants, or any other precipitates or supernatants from plasma fractionation, such as cryosupernatant, supernatant I+II+III, precipitate I+II+III, supernatant II+III, precipitate II+III, supernatant I+III, precipitate II, supernatant IV. Plasma fractions, precipitates and supernatants may be obtained from recovered plasma (plasma from whole blood) as well as from apheresis plasma. The volume of recovered plasma from one whole blood donations is usually between 100 and 240 ml, whereas the volume of a donation of apheresis plasma is usually between 500 and 800 ml.

A preferred viral inactivation method that can be carried out in the viral inactivation bag according to the invention is the S/D method (or solvent only as can be done for whole plasma or cryo-poor plasma), wherein the process chemicals that are added to the biological fluid are solvent/detergent combinations, or solvent alone.

Suitable solvents are di- or trialkylphosphates, such as tri-(n-butyl)phosphate, tri-(t-butyl)phosphate, tri-(n-hexyl)phosphate, tri-(2-ethylhexyl)phosphate, tri-(n-decyl)phosphate, di-(n-butyl)phosphate, di-(t-butyl)phosphate, di-(n-hexyl)phosphate, di-(2-ethylhexyl)phosphate, di-(n-decyl)phosphate as well as dialkylphosphates with different alkyl chains. Di- or trialkylphosphates having different alkyl chains can be employed, for example ethyl di(n-butyl) phosphate. An especially preferred trialkylphosphate is tri-(n-butyl)phosphate (TnBP).

Suitable detergents include polyoxyethylene derivatives of fatty acids, partial esters of sorbitol anhydrides, for example the products commercialized under the names "Tween 80" also known as "polysorbate 80" , "Tween 20", and non-ionic oil soluble water detergents, such as oxyethylated alkylphenol sold under the name "Triton X 100". Further contemplated detergents are sodium deoxycholate and sulfobetaines, such as N-dodecyl-N, N-dimethyl-2-ammonio-1ethane sulphonate. Especially preferred detergents are "Tween 80" and "Triton X-100".

The solvent and/or detergent can be extracted from the biological fluid by oil extraction with pharmaceutical grade oil. For this purpose the solvent and/or detergent containing biological fluid is preferably transferred into a funnel bag according to the present invention that holds sterile pharmaceutical grade oil.

One advantage of the funnel-like design of the lower portion of the inner compartment is the diminution of the contact surface between the oil and biological fluid phase towards the outlet facility and to a growth of the thickness of the upper phase towards the outlet facility. Therefore the interface (zone between the 2 mobile phases) is more and more obvious towards the end of the draining of the lower phase and the two phases can be easily separated, therefore optimizing removal of the viral inactivation chemicals and plasma volume recovery.

The pharmaceutical grade oil can be a naturally occurring oil, for example extracted from a plant or an animal, or a synthetic compound of similar structure. Suitable naturally occurring oils include castor oil (also known as ricinus oil), soybean oil, sunfflower oil, cottonseed oil. A preferred synthetic compound is a synthetic triglyceride. Examples of suitable synthetic triglycerides include triolein, tristearin, tripalmitin, trimyristin, and combinations thereof.

The amount of pharmaceutical grade oil which is present in the bag is the amount that allows the extraction of at least 80% of lipid soluble process chemicals, the oil being used in an amount from 2 to 20 weight% based on the weight of the biological fluid, preferably from 5 to 15 weight% and more preferably from 5 to 10 weight%.

Oil extraction may be carried out once, preferably twice and more preferably three times, depending in particular on the oil concentration.

The solvent and/or detergent may also be removed from the biological fluid by column chromatography using a funnel bag holding a stationary phase for column chromatography or a disposable chromatographic column device connected to the system according to the present invention, or other chromatographic system.

The column chromatography may be carried out subsequently to oil extraction or directly after solvent/detergent treatment.

The disposable viral inactivation bag according to the present invention can also be used for other viral inactivation methods of biological fluids using other process chemicals, for example methylene blue treatment, riboflavine (vitamin B2), or acid pH.

Viral inactivating treatment of biological fluids, especially plasma and plasma fractions, in a set of bags according to the invention allows for viral inactivation of small quantities of biological fluid.

This is particularly important, where the inactivation method is not effective against all types of viruses, as it is the case of the solvent/detergent treatment for non-enveloped viruses. In this way the risk of contamination of large pools of up to several thousands litres of biological fluid is avoided.

Another advantage of the treatment of small quantities is that a tailor-made production of virally inactivated biological fluid is possible. Accordingly a patient could for example be treated with virally inactivated plasma fractions coming exclusively from one donor, or small numbers of donors therefore decreasing the risks of exposure to any plasma-borne infectious agents.

Another advantage of the treatment as described in the present invention is the possibility to process and virally inactivate virally infected plasma, either as single donation, or as small pools, intended for therapeutic clinical studies, such as HCV positive plasma, or SARS positive plasma, or any other potentially infectious plasma that containing neutralizing antibodies susceptible to treat other patients. Accordingly a patient could for example be treated with virally inactivated HCV positive or SARS positive plasma fractions coming exclusively from one donor, or small numbers of donors therefore eliminating the risks of exposure to HCV or SARS or any plasma-borne infectious agents.

The above and other objects, features and advantages of the present invention will become more apparent from the following description, reference being made to the accompanying figures, in which
Figure 1 is a schematic cross-sectional view of one embodiment of a viral inactivation bag according to the present invention;
Figure 1a is a schematic cross sectional view along the line AA' of the embodiment of figure 1 when the bag is empty;
Figure 1b is a schematic cross sectional view along the line AA' of the embodiment of figure 1 when he bag is filled;
Figure 2 is a schematic cross-sectional view along of one embodiment of an extraction bag according to the present invention;
Figure 2a is a schematic cross-sectional view along the line AA' of the embodiment of figure 2 when the bag is empty;
Figure 2b is a schematic cross-sectional view along the line AA' of the embodiment of figure 2 when the bag is filled;
Figure 3 is a schematic cross-sectional view of one embodiment of a disposable chromatographic column;
Figure 4 is a schematic cross-sectional view of one embodiment of the set of disposable bags according to the present invention.

The terms horizontal, vertical, upper, and lower are used hereafter with respect to the position of the bags in use where the outlet facility is directed vertically downwards.

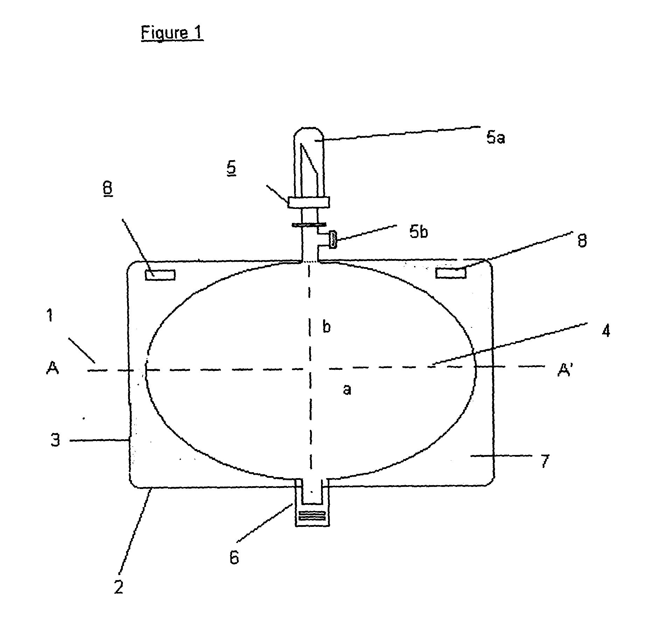

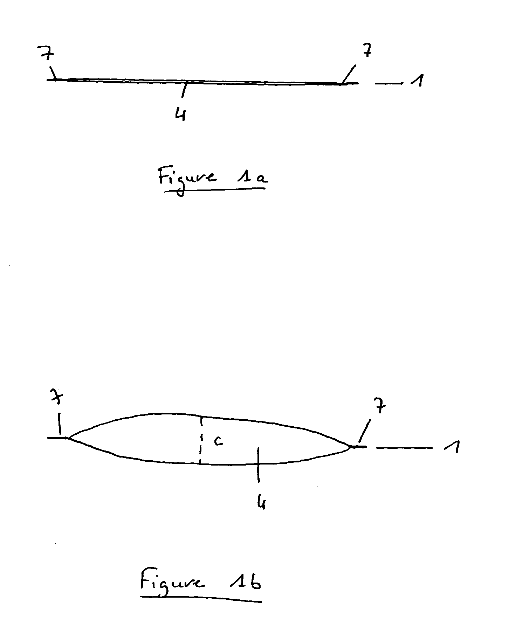

The viral inactivation bag 1 illustrated in figure 1 has an essentially rectangular outer shape, the horizontal sides 2 being longer than the vertical ones 3.

The bag 1 comprises an inner compartment 4, an inlet facility 5 and an outlet facility 6. The inner compartment 4 has a cross-section in the form of an ellipse with a first axis a, and a second axis b. The first axis a is parallel to the horizontal sides 2 of the bag 1, and the second axis b is parallel to its vertical sides 3, with a ratio a/b >1.

The inlet 5 and outlet 6 facilities are each separately connected to the inner compartment 4. The inlet facility 5 is arranged in the middle of the upper horizontal side 3 and coincides with the second axis b of the ellipse. The outlet facility 6 is arranged directly opposed to the inlet facility 5 in the middle of the lower horizontal side 3 and coincides as well with the second axis b of the ellipse.

The inlet facility 5 comprises a flexible tubular inlet line, a first port 5a being connected to the end of the inlet line and a second port 5b being laterally connected to the inlet line. The first port 5a is a bag spike. It is employed to transfer biological fluid from, for example, a storage or collection bag into the inner compartment 4 of the viral inactivation bag 1. The second port 5b is a port for the addition of process chemicals.

The viral inactivation bag 1 is made of two rectangular sheets of conventional medical/pharmaceutical grade polyvinylchloride (PVC) or other adequate material that are laminated and welded together on their periphery so that the inner compartment 4 comprising two apertures for the inlet 5 and outlet 6 facilities respectively is formed.

The inner compartment 4 is surrounded by a welded together border area 7 that extends to the limits of the sheets. This border area 7 comprises two holes 8, that are located left and right of the inlet facility. The holes 8 serve as means for suspending the bag 1 vertically.

Figure 1a illustrates an empty viral inactivation bag 1. In figure 1b the same bag is filled and the sheets are separated and bulged outwardly. The thickness c of the filled bag is smaller than the smallest of the two axis a and b.

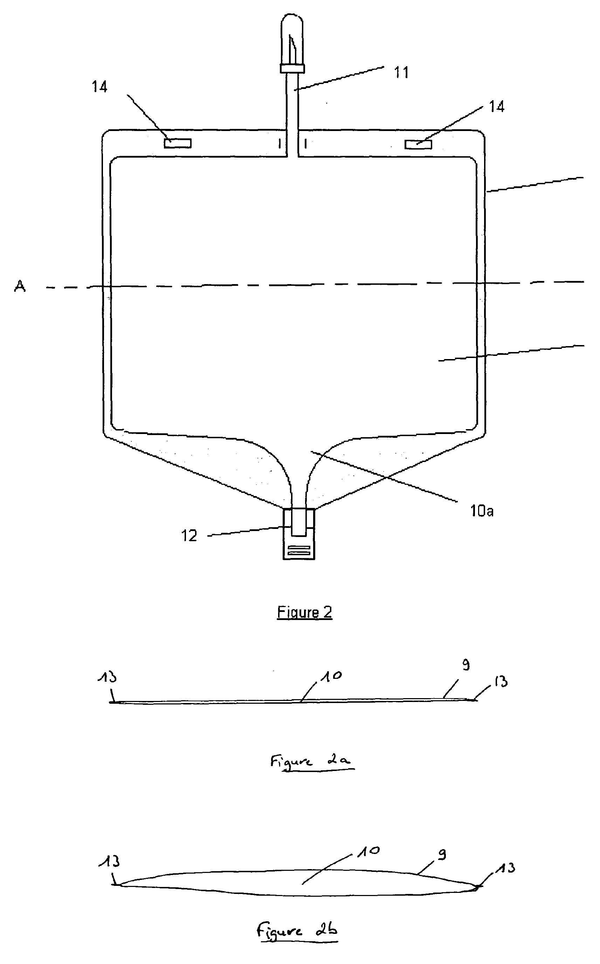

The funnel bag 7 illustrated in figure 2 comprises an inner compartment 8, an inlet facility 9 and an outlet facility 10. The inlet 9 and outlet 10 facilities are each separately connected to the elliptic inner compartment 8 and are arranged on opposite sides of the bag 7.

The inlet facility comprises a flexible tubular inlet line connected at one end to the inner compartment 8 and at the other end to a state of the art plasma bag spike. In this way, the process chemicals containing biological fluid can easily be transferred into the extraction bag by gravity from, for example, a viral inactivation bag 1.

The funnel bag 1 is made of two sheets of conventional medical/pharmaceutical grade polyvinylchloride (PVC) or other adequate material that are laminated and welded together on their periphery so that the inner compartment 10 comprising two apertures for the inlet 11 and outlet 12 facilities respectively is formed.

The inner compartment 10 is surrounded by a welded together border area 13 that extends to the limits of the sheets. This border area 13 comprises two holes 14, that are located left and right of the inlet facility. The holes 14 serve as means for suspending the bag 9 vertically.

Figure 2a illustrates an empty funnel bag 9. In figure 2b the same bag is filled and the sheets are separated and bulged outwardly.

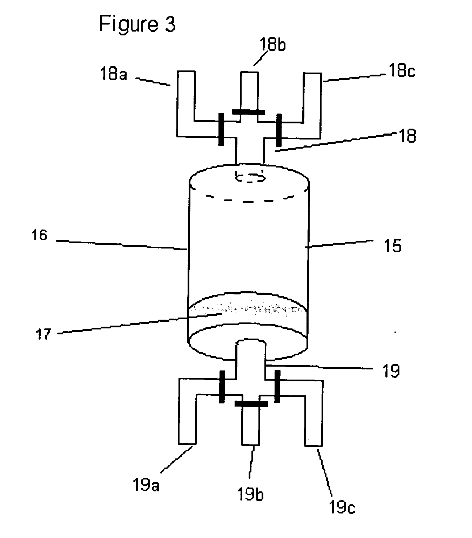

The disposable chromatographic column bag device 15 represented in figure 3 comprises a cylindrical hollow body 16 filled with a stationary chromatographic phase, the lower part of the hollow body is provided with a filter 17. The cylindrical body is provided with an inlet facility 18 in its higher end and with an outlet facility 19 at its opposed lower end. The inlet facility comprises three ports (18a, 18b, 18c), the outlet facility also comprises three ports (19a, 19b and 19c).

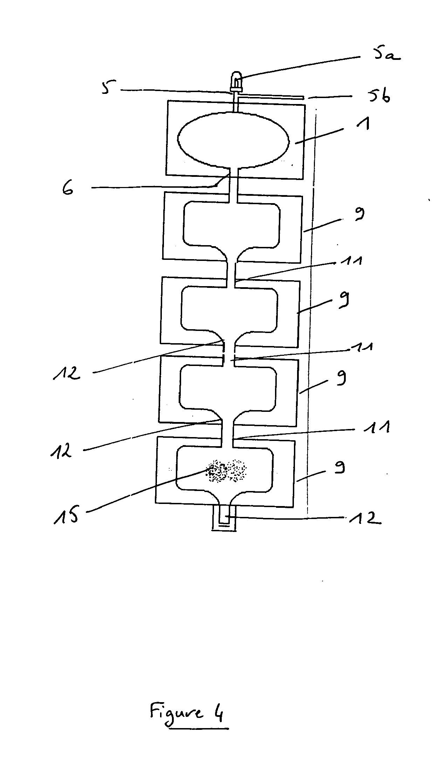

The set of disposable bags illustrated in figure 4 comprises a viral inactivation bag 1 according to the invention, three subsequent funnel bags 9 according to the invention pre-filled with pharmaceutical grade oil (the oil not being represented), and a funnel bag 9 according to the invention pre-filled with a stationary phase for column chromatography.

The bags are connected to each other with flexible tubes, i.e. the outlet facility 6 of the viral inactivation bag 1 is connected to the inlet facility 11 of the first funnel bag 9, the outlet facility 12 of the first bag 9 is connected to the inlet facility 11 of the second funnel bag 9, the outlet facility 12 of the second funnel bag 9 to the inlet facility 11 of the third funnel bag 9 and the outlet facility 12 of the third funnel bag 9 to the inlet facility 11 of the stationary phase containing funnel bag 9. The outlet facility 12 of the stationary phase containing funnel bag 9 is sealed.

In order to more fully illustrate the nature of the invention and the manner of practising the same, the following non-limiting examples are presented:

### EXAMPLES

### EXAMPLE 1: Viral inactivation of 1915 ml apheresis plasma using 2% TnBP

Apheresis plasma from three donations of about 650 ml is transferred into a sterile, single 2000 ml viral inactivation bag according to figure 1 and the bag is heated to 31°C +/- 1°C.

A solution of pure tri(n-butyl)phosphate (TnBP) is pre-filled into a sterile syringe.

Then the TnBP solution is added with the syringe (via the second port of the inactivation bag) slowly over 30 minutes to the plasma. The inactivation bag is under gentle shaking, such as using a platelet concentrate shaker device until a final concentration of 2% of the total plasma weight was reached.

After the addition of the TnBP solution the tubing of the inlet facility is heat-sealed in order to isolate the inactivation bag.

Then, vigorous mixing is performed for 30 sec, and the bag is maintained at 31°C +/- 1°C for 4 hrs under gentle stirring such as using a platelet concentrate shaker device or any other appropriate device ensuring mild constant mixing of the plasma/SD mixture.

The plasma is then transferred into a funnel bag according to figure 2 containing 100 ml pharmaceutical grade castor oil. For this purpose the outlet facility of the viral inactivation bag is pierced with the spike of the inlet facility of the funnel bag and the TnBP containing plasma is transferred by gravity.

The oil/plasma mixture is shaken vigorously until the formation of an emulsion for about one minute, then gently for 15 minutes at room temperature in order to extract TnBP with the oil.

The funnel bag is then hung up in order to allow for separation of a lower plasma phase and an upper TnBP containing oil phase.

The lower layer is drained by gravity through the outlet facility into a second funnel bag containing 150 ml of pharmaceutical grade castor oil. The phase interface is detected with a UV detector that is arranged on the level of the outlet facility of the first funnel bag.

The oil extraction is repeated twice.

Factor VIII activity, Factor IX activity, and global coagulation tests PT and PTT are performed in the plasma after the various steps:

| | Plasma | Plasma + 2% TnBP | 1 oil | 2 oil | 3 oil |
|---|---|---|---|---|---|
| PT, sec | 15,1 | 20,7 | 16,7 | 14,7 | 14,3 |
| PTT, sec | 41,9 | 66,7 | 40,9 | 41,1 | 43,1 |
| FVIII, % | 85 | 67 | 54 | 72 | 86 |
| FIX, % | 75 | 53 | 90 | 86 | 88 |

Results show that global coagulant activity of plasma as assessed by PT and PTT is preserved when comparing the plasma after 3 oil extractions to the starting plasma. Similarly the recovery of FVIII and FIX is excellent.

Data also show that the PT and PTT are prolonged when plasma contains 2% TnBP and normalized after 2 oil extractions, illustrating the removal of the TnBP.

### EXAMPLE 2: Viral inactivation of 200 ml recovered plasma

Recovered plasma from one donation of about 200 ml is transferred into a sterile, single 200 ml viral inactivation bag according to figure 1 and the bag is heated to 31°C +/- 1°C.

A solution of pure tri(n-butyl)phosphate (TnBP) is pre-filled into a sterile syringe.

Then the TnBP solution is added with the syringe (via the second port of the inactivation bag) slowly over 30 minutes to the plasma. The inactivation bag is under gentle shaking, such as using a platelet concentrate shaker device until a final concentration of 2% of the total plasma weight is reached.

After the addition of the TnBP solution the tubing of the inlet facility is heat-sealed in order to isolate the inactivation bag.

Then vigorous mixing is performed for 30 sec, and the bag is maintained at 31°C +/- 1°C for 4 hrs under gentle stirring such as using a platelet concentrate shaker device or any other appropriate device ensuring mild constant mixing of the plasma/SD mixture

The plasma is then transferred into a funnel bag according to figure 2 containing 10 ml pharmaceutical grade castor oil. For this purpose the outlet facility of the viral inactivation bag is pierced with the spike of the inlet facility of the funnel bag and the TnBP containing plasma is transferred by gravity.

The oil/plasma mixture is shaken vigorously until the formation of an emulsion for about one minute, then gently for 15 minutes at room temperature in order to extract TnBP using the oil.

The funnel bag is then hung up in order to allow for separation of a lower plasma phase and an upper TnBP containing oil phase.

The lower layer is drained by gravity through the outlet facility into a second funnel bag containing 150 ml of pharmaceutical grade castor oil. The phase interface is detected with a UV detector that is arranged on the level of the outlet facility of the first funnel bag.

The oil extraction is repeated twice.

The TnBP is measured in the plasma after each extraction step:

| **Step** | **TnBP content measured by Gas chromatography** |
|---|---|
| Plasma with 2% TnBP | 2% |
| Plasma after 1 oil extraction | 1.2% |
| Plasma after 2 oil extractions | 320ppm |
| Plasma after 3 oil extractions | Undetectable (< 10 ppm) |

Results show that TnBP is not detectable (< 10 ppm) in the final plasma after 3 oil extractions.

The PT, PTT, INR (international normalized ratio), FVIII and FIX are also determined at all steps:

| | Plasma | SD | 1 oil | 2 oil | 3 oil |
|---|---|---|---|---|---|
| PT, sec | 13,7 | 20,9 | 16,1 | 14,7 | 14,7 |
| INR | 1,31 | 2,68 | 1,72 | 1,47 | 1,47 |
| PTT, sec | 40,1 | 74,4 | 41,9 | 41,9 | 40,7 |
| FVIII, % | 70,7 | 89,9 | 84,9 | 70,7 | 81 |
| FIX, % | 83,3 | 81 | 130 | 87,3 | 76,5 |

Results show the good recovery in coagulant activity achieved as well as the normalisation of the value of all assays after 3 oil extractions.

**EXAMPLE 3:** Recovery of protein C, Protein S, alpha 2 antiplasmin, and Von Willebrand factor ristocetin cofactor activity in 200ml apheresis plasma virally inactivated with 2% TnBP

Apheresis plasma from one donation of about 200 ml is transferred into a sterile, single 200 ml viral inactivation bag according to figure 1 and the bag is heated to 31°C +/- 1°C.

A solution of pure tri(n-butyl)phosphate (TnBP) is prefilled into a sterile syringe.

Then the TnBP solution is added with the syringe (via the second port of the inactivation bag) slowly over 30 minutes to the plasma. The viral inactivation bag is under gentle shaking, such as using a platelet concentrate shaker device of the bag until a final concentration of 2% of the total plasma weight is reached.

After the addition of the TnBP solution the tubing of the inlet facility is heat sealed in order to isolate the inactivation bag.

Then vigorous mixing is performed for 30 sec, and the bag is maintained at 31°C +/- 1°C for 4 hrs under gentle stirring such as using a platelet concentrate shaker device or any other appropriate device ensuring mild constant mixing of the plasma/SD mixture

The plasma is then transferred into a funnel bag according to figure 2 containing 10 ml pharmaceutical grade castor oil. For this purpose the outlet facility of the viral inactivation bag is pierced with the spike of the inlet facility of the funnel bag and the TnBP containing plasma is transferred by gravity.

The oil/plasma mixture is shaken vigorously until the formation of an emulsion for about one minute, then gently for 15 minutes at room temperature in order to extract TnBP using the oil.

The funnel bag is then hung up in order to allow for separation of a lower plasma phase and an upper TnBP containing oil phase.

The lower layer is drained by gravity through the outlet facility into a second funnel bag containing 150 ml of pharmaceutical grade castor oil. The phase interface is detected with a UV detector that is arranged on the level of the outlet facility of the first funnel bag.

The oil extraction is repeated twice.

The values of Protein C, Protein S, alpha 2 antiplasmin, and von willebrand factor ristocetin cofactor activity are determined at all steps:

| | Plasma | SD | 1 oil | 2 oil | 3 oil |
|---|---|---|---|---|---|
| Protein C | 74.1 | 59.4 | 66.2 | 66.7 | 60.8 |
| Protein S | 50.6 | 16.8 | 51.8 | 47.7 | 49.5 |
| Alpha 2 antiplasmin | 122.9 | 178.7 | 133.7 | 136.6 | 140.6 |
| Von Willebrand Factor, Ristocetin cofactor | 84 | 84 | 112 | 84 | 84 |

Results show the good recovery in the activity achieved after 3 oil extractions.

## Claims

1. Set system of disposable bags comprising a viral inactivation bag (1), comprising an inner compartment (4), an inlet facility (5) and an outlet facility (6), which are both connected to the inner compartment (4), the bag (1) being **characterised in that** the inner compartment (4) has an ovoid cross section, and optionally, at least one funnel bag (9), and/or a column chromatography bag (15), the different bags being connectable with each other.

2. The set system of disposable bags according to claim 1 **characterised in that** the cross section of the inner compartment (4) is elliptic.

3. The set system of disposable bags according to claim 1 or 2, **characterised in that** the volume of the inner compartment (4) is in the range from 50 ml to 5000 ml, preferably from 100ml to 3000ml and even more preferably from 200 to 2000ml.

4. The set system of disposable bags according to any of claims 1 to 3, **characterised in that** the funnel extraction bag (9) comprise an inner compartment (10), an inlet facility (11) and an outlet facility (12), which are both connected to the inner compartment (10), the inner compartment (10) having a lower portion in the form of a funnel that meets the outlet facility (12) and the inner compartment (10) containing a separation agent.

5. The set system of disposable bags according to any of claims 1 to 4, **characterised in that** the bags (1, 9, 15) are made of medical/pharmaceutical grade polyvinylchloride.

6. The set system of disposable bags according to any of claims 1 to 5, **characterised in that** it comprises three subsequent funnel bags (9), wherein the separation agent is pharmaceutical grade oil, and one extra funnel bag (9), wherein the separation agent is a stationary phase for column chromatography.

7. The set system of disposable bags according to any of claims 1 to 6, **characterised in that** the bags are connected with flexible tubes and that the outlet facility (6) of the viral inactivation bag (1) is connected to the inlet facility (11) of the first funnel bag (9), the outlet facility (12 of the first bag (9) is connected to the inlet facility (11) of the second funnel bag (9), the outlet facility (12) of the second funnel bag (9) is connected to the inlet facility (11) of the third funnel bag (9) and the outlet facility (12) of the third funnel bag (9) is connected to the inlet facility (11) of the stationary phase containing funnel bag (9).

8. The set system of disposable bags according to claims 1 to 7, **characterised in that** the bags are connected in such a way to ensure sterility of the biological fluid to be treated.

9. Disposable viral inactivation bag (1), comprising an inner compartment (4), an inlet facility (5) and an outlet facility (6), which are both connected to the inner compartment (6), the bag (1) being
**characterised in that** the inner compartment (4) has an ovoid cross section.

10. Disposable funnel bag (9) comprising an inner compartment (10), an inlet facility (11) and an outlet facility (12), which are both connected to the inner compartment (10), the inner compartment (10) having a lower portion in the form of a funnel that meets the outlet facility (12) **characterised in that** the inner compartment (10) holds a separation agent.

11. Method of viral inactivation of a biological fluid comprising the steps of
a. viral inactivation of the biological fluid in a viral inactivation bag according to claim 9, and optionally
b. oil extraction of the biological fluid in a pharmaceutical grade oil containing funnel bag according to claim 10, and/or
c. chromatography of the biological fluid in a column chromatography bag.

12. Use of the set system of disposable bags according to any of claims 1 to 8 for the viral inactivation of biological fluids with excellent protein recovery.
